# EUROPÄISCHE PATENTSCHRIFT

(11) **EP 1 232 277 B1**
(45) Veröffentlichungstag und Bekanntmachung des Hinweises auf die Patenterteilung: **28.05.2003**
(21) Anmeldenummer: 00989882.6
(22) Anmeldetag: 24.11.2000
(51) Int. Cl.: C12P 13/00, C12N 9/88, C12N 11/00

(54) **VERFAHREN ZUR HERSTELLUNG VON OPTISCH AKTIVEN CYANHYDRINEN**
METHOD FOR PRODUCING OPTICALLY ACTIVE CYANHYDRINS
PROCEDE DE PRODUCTION DE CYANHYDRINES OPTIQUEMENT ACTIVES

(30) Priorität: 25.11.1999 DE 19956864; 24.11.2000 DE 10058342
(43) Veröffentlichungstag der Anmeldung: 21.08.2002
(73) Patentinhaber: Degussa AG, 40474 Düsseldorf (DE)
(72) Erfinder: GRÖGER, Harald, 83308 Trostberg (DE); VORLOP, Klaus-Dieter, 38102 Braunschweig (DE); CAPAN, Emine, 31226 Peine (DE)
(74) Vertreter: Retzow, Stefan
(86) Internationale Anmeldenummer: EP0011743
(87) Internationale Veröffentlichungsnummer: WO01038554

(56) Entgegenhaltungen:
- US-A- 5 177 242
- FADNAVIS NITIN W ET AL: "An unusual reversible sol-gel transition phenomenon in organogels and its application for enzyme immobilization in gelatin membranes." BIOTECHNOLOGY PROGRESS, Bd. 15, Nr. 1, Januar 1999 (1999-01), Seiten 98-104, XP001009849 ISSN: 8756-7938
- JEKEL, MAREN ET AL.: "Immobilization of Biocatalysts in LentiKats" CHEMICAL ENGINEERING AND TECHNOLOGY, Bd. 21, Nr. 3, 1998, Seiten 275-278, XP001009940
- JIMENEZ C ET AL: "Use of photopolymerizable membranes based on polyacrylamide hydrogels for enzymatic microsensor construction." ANALYTICA CHIMICA ACTA, Bd. 351, Nr. 1-3, 1997, Seiten 169-176, XP001010096 ISSN: 0003-2670
- GROEGER, HARALD ET AL: "Asymmetric Synthesis of an (R)-Cyanohydrin Using Enzymes Entrapped in Lens-Shaped Gels" ORG. LETT. (2001), 3(13), 1969-1972, XP002171018

## Beschreibung

Die Erfindung betrifft ein Verfahren zur Herstellung von optisch aktiven Cyanhydrinen.

Optisch aktive Cyanhydrine sind von beträchtlichem Interesse bei der Herstellung von optisch aktiven Aminoalkoholen, α-Hydroxycarbonsäuren, sowie Heterozyklen und Pyrethroidinsektiziden. Von grundlegender Bedeutung ist dabei eine ökonomische Herstellung der Zielmoleküle in hohen Ausbeuten und mit großer Enantioselektivität sowie unter Minimierung der Herstellungskosten, insbesondere der für diesen Prozess wichtigen Enzymkosten.

Die Herstellung optisch aktiver (R)-Cyanhydrine durch Umsetzung von Blausäure mit einem Aldehyd in Gegenwart von (R)-Oxynitrilase als Enzymquelle wurde beispielsweise von Effenberger et al. beschrieben (F. Effenberger et al., Angew. Chem. 1987, 99, 491-492). Diese Reaktion findet im 2-Phasensystem bestehend aus einer organischen, mit Wasser nicht mischbaren Lösemittel-Phase, vorzugsweise Essigsäureethylester, sowie einer wässrigen Phase statt. Die Umsetzung erfolgt dabei zumindest für einen Teil der Aldehyde mit ausgezeichneten Ausbeuten und optischen Reinheiten. In Bezug auf die optische Reinheit der Cyanhydrine wurde die enzymatische Addition von Cyaniddonoren an Aldehyde in Gegenwart der Enzyme (R)-Oxynitrilase bzw. (S)-Oxynitrilase bereits eingehend untersucht. Die optische Reinheit des Cyanhydrin-Produkts hängt dabei in erster Linie davon ab, in welchem Maße die unerwünschte Nebenreaktion der racemischen Cyanhydrinbildung unterdrückt werden kann.

Dem technischen Einsatz der isolierten Oxynitrilasen bzw. der biologisch aktiven isolierten Enzyme stehen allerdings im Allgemeinen ihr oftmals hoher Preis, ihre geringe Stabilität und Langzeitaktivität sowie die Schwierigkeiten bei der Aufarbeitung und Wiederverwendung hindernd im Wege.

Diese Nachteile werden vielfach durch die Bindung der Enzyme an einen unlöslichen Träger beseitigt. So ermöglicht die Trägerfixierung eine vielfache Wiedergewinnung und macht so den Einsatz der Enzyme in technischen Anwendungen überhaupt erst möglich.

Bereits sehr früh wurden die Vorteile einer Immobilisierung auch für das Enzym (R)-Oxynitrilase erkannt. So wird in der DE-PS 13 00 111 die Herstellung von chiralen Cyanhydrinen unter Verwendung einer an ein Ionenaustauscherharz gebundenen Oxynitrilase beschrieben. Die Werte für die Enantioselektivitäten lagen allerdings unter 90 %.

Eine Verbesserung der Enantioselektivitäten wurde von Effenberger et al. erzielt, indem die Cyanhydrinsynthese in Gegenwart einer an einen Träger immobilisierten (R)-Oxynitrilase in Essigsäureethylester bei pH-Werten von 5,4 durchgeführt wurde. Allerdings wird dadurch die Enzymstabilität, wie generell in organischen Medien, stark vermindert (P. Methe et al., US-PS 5,122,462). Über eine geringe Langzeitstabilität wurde zudem von Gil et al. berichtet (J. Am. Chem. Soc., 1999, 120, 8587). Demnach liegt der Aktivitätsverlust nach 322 h bei 87 %!

Aus dem US-Patent 4,859,784 ist bekannt, dass die Enzymimmobilisierung durch Bindung der (R)-Oxynitrilase an Glasoberflächen, Resin-Ionenaustauscherharzen oder an Cellulose-Partikel erfolgen kann. Allerdings wird vom Dokument US 5,177,242 dargelegt, dass bei Verwendung dieser so immobilisierten (R)-Oxynitrilasen der Durchsatz der Reaktanden einen sehr langen Zeitraum von 5 bis 6 Tagen benötigt.

Darüber hinaus sind noch eine Reihe weiterer Methoden zur Immobilisierung von Oxynitrilasen entwickelt worden: So besteht eine Möglichkeit der Immobilisierung in der Einbindung von (R)-Oxynitrilasen in Flüssigkristalle, die dann in einem Mehrphasen-Prozess zur Synthese chiraler Cyanhydrine eingesetzt werden; diese Reaktion wird bevorzugt in einem kontinuierlichen Reaktor durchgeführt (US-PS 5,122,462).

Über Sol-Gel-immobilisierte Oxynitrilasen wurde ebenfalls berichtet. Mit den dabei entwickelten Gelatin- oder Si-basierenden Methoden werden allerdings die Trägerimmobilisate in leicht brüchiger Pulverform und somit in nicht-definierter makroskopischer Größe erhalten. Bei den Gelatin/Gel-immobilisierten Enzymen, deren Hauptpartikelgröße sich zwischen 0,3 und 0,5 mm bewegt, was somit zu Problemen bei der Filtration führt, kann zudem nicht im wässrigen System gearbeitet werden (Biotechnol. Prog. 1999, 15, 98 - 104). Einige der Probleme bei den Si-basierenden Immobilisaten stellen die nicht definierte makroskopische Größe (einhergehend mit Filtrationsschwierigkeiten), die hohe Porosität, eine geringe Elastizität sowie die ökonomisch aufwendige Herstellung dar. Auch über einen Aktivitätsverlust von 16 % nach 322 h wurde berichtet (J. Am. Chem. Soc., 1999, 120, 8587).

Ergänzend wurden ebenfalls Methoden zur Immobilisierung der (S)-Oxynitrilasen entwickelt, die von der Funktionsweise denen der (R)-Oxynitrilasen vergleichbar sind. So wird von Effenberger et al. die Immobilisierung der (S)-Oxynitrilasen durch die Anbindung an einen Nitrocelluloseträger erreicht (F. Effenberger et al., Angew. Chem. 1996, 108, 493-494). Über eine Immobilisierung durch Anbindung des Enzyms an eine poröse Membran berichten Andruski et al. (US 5,177,242).

Trotz dieser z. T. durchaus erfolgversprechenden Ansätze mit immobilisierten Enzymen sind neuerdings wieder vermehrt Berichte erschienen, die von Arbeiten mit nicht-immobiliserten Enzymen berichten (bspw. EP-A 0 927 766 und US 5,714,356). Stehen diese Entwicklung doch im krassen Gegensatz zu den ohne Zweifel prinzipiell vorhandenen Vorteilen, die mit trägerimmobilisierten Oxynitrilasen verbunden sind, so dürfte der Grund dafür in den bislang noch immer nicht überwundenen Nachteilen und ungelösten Problemen bei der Herstellung und Anwendung von trägerimmobilisierten Oxynitrilasen liegen.

Ein beträchtlicher Nachteil des Standes der Technik liegt generell in einer nicht zufriedenstellenden Langzeitstabilität: So wird beispielsweise bei Wehtje et. al über einen typischen Immobilisierungseffekt berichtet, der sich in einem Verlust von 40 % nach 200 h Reaktionszeit äußert (Appl. Microbiol. Biotechn. 1988, 29, 419).

Ein erheblicher genereller Nachteil bisheriger trägerimmobilisierter Oxynitrilasen ist deren im Allgemeinen geringe Abriebfestigkeit und eingeschränkte mechanische Stabilität in technischen Reaktoren. Gerade in technischen Reaktoren erfolgt nämlich eine starke Beanspruchung des immobilisierten Systems durch heftiges Rühren (in Batch-Reaktoren). Auch in kontinuierlich betriebenen Reaktoren wird das immobilisierte System z. B. durch Scherkräfte stark beansprucht. Zur Problematik eines unerwünschten Abriebs in mechanisch stark beanspruchten Reaktorsystemen kommt zudem bei den bislang entwickelten Immobilisierungstechniken für Oxynitrilasen noch die Problematik eines "Katalysatorleachings", also eines Verlusts des Katalysators und damit der katalytischen Aktivität während der Reaktion, hinzu. Neben der beschränkten mechanischen Stabilität der bisher entwickelten immobilisierten Systeme liegt ein weiterer Nachteil in der zumeist aufwendigen und zudem teuren Immobilisierungstechnik (z.B. Flüssigkristalle, siloxanimmobilisert mit mehreren Komponenten).

Ein weiteres Problem ist die Abtrennung des Trägers durch Filtration: Typische Trägerimmobilisate sind pulvrig porös mit einem breiten Spektrum bzgl. Partikelgröße, wobei typische Partikelgrößen von 0,3 bis 0,7 mm bei der Filtration zu erheblichen Problemen im technischen Maßstab führen. Wünschenswert wären somit Immobilisate mit definierter Größe und einer Partikelgröße von > 1 mm, um eine technisch günstige Filtration zu ermöglichen.

Aus den genannten Nachteilen hat sich deshalb die Aufgabe gestellt, ein Verfahren zur Herstellung von optisch aktiven Cyanhydrinen durch Umsetzung von Oxoverbindungen mit Blausäure und/oder mit einem Cyaniddonor und in Gegenwart einer Einschlussverbindung, die eine molekulargewichtsvergrößerte Oxynitrilase enthält, zu entwickeln, das die beschriebenen Nachteile immobilisierter Enzyme nicht aufweist und möglichst folgende Vorteile aufweisen sollte:
- Minimale Kosten der Immobilisierungstechnik
- Einfache Durchführung der Immobilisierung
- Hohe mechanische Stabilität und große Elastizität des immobilisierten Systems
- Hohe Stabilität und Aktivität der Enzyme
- Vernachlässigbares "Leaching" der Enzymquelle
- Einfache Abtrennung makroskopischer (> 1 mm) Enzym-Komponenten
- Hohe "Turnover Numbers" (TON) der Cyanhydrin-Synthese durch lange Wiederverwendbarkeit des enzymatischen Systems
- Definierte Partikelgröße des immobilisierten Systems.

Die Aufgabe wurde erfmdungsgemäß durch das in Anspruch 1 angegebene Verfahren gelöst, wobei man eine Oxynitrilase verwendet, die im wesentlichen in zwei voneinander unabhängigen Schritten erstens durch Quervernetzung molekularvergrößert und anschließend in eine Gel-Einschluss- und/oder eine Gel-Komplex-Verbindung überführt worden ist.

Überraschenderweise wurde gefunden, dass die eingeschlossenen und/oder komplexierten Enzyme eine hohe Aktivität aufweisen. Außerdem besitzen die im Gel eingeschlossenen Enzyme ein hohes Maß an Stabilität. Dies war nicht zwangsläufig zu erwarten, da die Immobilisierung im Allgemeinen mit Aktivitätsverlusten und Stabilitätsproblemen verbunden ist. Des Weiteren ist als überraschend anzusehen, dass an diesen Oxynitrilasen-Varianten nahezu kein Abrieb beobachtbar ist. Die als Kapseln zu bezeichnenden und in einer elastischen Gelmatrix eingeschlossenen Enzyme sind während der großtechnischen Anwendung über einen mehrwöchigen bis mehrmonatigen Zeitraum völlig stabil und abriebsfest, was gleichbedeutend ist mit einer bislang unerreichten hohen Stabilität eines immobilisierten Oxynitrilase-Systems. Überraschend ist zudem, dass Leaching-Verluste der Enzym-haltigen Komponente praktisch nicht vorhanden, also vernachlässigbar klein sind.

Das erfindungsgemäße Verfahren zur Herstellung von optisch aktiven Cyanhydrinen wird vorzugsweise bei Reaktionstemperaturen von - 10 bis + 30 °C und insbesondere von 5 bis + 25 °C durchgeführt, wobei erfindungsgemäß der pH-Wert vorzugsweise zwischen 3,0 und 6,0 liegen sollte.

In einer bevorzugten Ausführungsform der Erfindung wird das Herstellungsverfahren in einem wässrigen oder wasserhaltigen System ausgeführt, das in der Regel als Lösemittel fungiert. Als besonders geeignet haben sich hierbei Wasser oder beliebige Mischungen aus Wasser und Ethanol, Methanol, Essigsäureethylester, Diisopropylether, Methyl-tert-butylether und Hexan gezeigt. Die wasserhaltigen Systeme weisen bevorzugt einen Wassergehalt von mindestens 25 Gew.-%, bevorzugt mindestens 40 Gew.-% auf.

Aber auch organische Lösemittel, vorzugsweise Methanol, Ethanol, Essigsäureethylester, Diisopropylether, Methyl-tert-butylether, Toluol und chlorierte Lösemittel wie z. B. Dichlormethan sind für die Umsetzung bestens geeignet. In einer weiteren bevorzugten Ausführungsform der Erfindung wird das Herstellungsverfahren deshalb in mindestens einem organischen Lösungsmittel durchgeführt, wobei hier das System praktisch wasserfrei sein kann und bevorzugt weniger als 20 Gew.-% und insbesondere weniger als 10 Gew.-% Wasser enthält.

Ebenso unkritisch wie das Lösemittel sind die stöchiometrischen Verhältnisse der Reaktionspartner anzusehen, so dass sie aus einem großen Bereich ausgewählt werden können. Besonders vorteilhaft sind jedoch stöchiometrische Verhältnisse der Oxoverbindung zur Blausäure und/oder dem Cyaniddonor, die zwischen 1 : 1 und 1 : 10 und vorzugsweise zwischen 1 : 1 und 1 : 4,0 liegen.

Der Begriff "Oxoverbindungen" bezeichnet Verbindungen, die ein doppelt gebundenes Sauerstoffatom (=O) aufweisen. Als Oxoverbindungen kommen gemäß Erfindung insbesondere Aldehyde und/oder Ketone zum Einsatz, in denen der Sauerstoff an ein Kohlenstoffatom gebunden ist. Da optisch aktive Cyanhydrine ein asymmetrisches C-Atom (Chiralitätszentrum) besitzen, werden bevorzugt asymmetrisch substituierte Oxoverbindungen der Formel R¹-C(O)-R² eingesetzt, in denen die Reste R¹ und R² jeweils unabhängig H, eine Alkylgruppe, welche geradkettig oder verzweigt sein kann, insbesondere eine C₁-C₂₀-Alkylgruppe oder eine Arylgruppe, insbesondere eine C₆-C₂₀-Arylgruppe bedeuten, wobei die Alkyl- oder/und Arylgruppen substituiert sein können, z.B. mit Halogen, wie etwa F, Cl, Br oder J, Alkoxy, z.B. C₁-C₄-Alkoxy, Phenoxy oder mit Alkyl- oder Arylgruppen, mit der Maßgabe, dass R¹ ≠ R².

Die Verwendung solcher Oxoverbindungen führt z.B. zur Bildung von optisch aktiven Cyanhydrinen mit der Formel R¹R² C (OH) (CN). Bevorzugte Oxoverbindungen sind aromatische C₆₋₂₀-Aldehyde, aliphatische C₁₋₂₀-Aldehyde, wie vorzugsweise Benzaldehyd oder substituierte und insbesondere mit Alkoxy-, Phenoxy-, Alkyl-, Aryl- oder Halogen-substituierte Benzaldehyde sowie mit 2 unterschiedlichen Alkyl- und/oder Arylresten substituierte C₄₋₂₀-Ketone, wie z.B. 2-Butanon, 2-Pentanon oder Acetophenon.

Als Cyaniddonor kann jede Verbindung eingesetzt werden, die bei der Umsetzung eine Cyanidgruppe liefert. Bevorzugt wird als Cyaniddonor und Blausäurequelle die Blausäure selbst oder vorzugsweise Acetoncyanhydrin und/oder Trimethylsilylcyanid verwendet. Selbstverständlich sind aber auch Cyanidsalze, wie beispielsweise Natriumcyanid oder Kaliumcyanid, für das vorliegende Verfahren bestens geeignet.

Insgesamt ist die Reaktionsführung als völlig problemlos einzuordnen. Bei Verwendung von Wasser als Reaktionsmedium wird die Reaktion vorteilhaft im gepufferten sauren Medium bei pH-Werten von 3,0 bis 4,0 durchgeführt. Am geeignetsten haben sich milde Reaktionstemperaturen erwiesen, wobei das vorliegende Verfahren bei Raumtemperatur offensichtlich am besten gelingt.

Alternativ kann die Reaktion aber auch, wie beschrieben, in einem wässrigorganischen oder ausschließlich organischen Reaktionssystem durchgeführt werden. Hier gelingt die Reaktion besonders effizient bei pH-Werten von 3,0 bis 6,0 und vor allem zwischen 4,0 bis 5,0.

In bestimmten Verfahrensvarianten hat es sich als günstig erwiesen, wenn das erfindungsgemäße Verfahren vorzugsweise in einem System durchgeführt wird, in dem pro Gramm Oxoverbindung 0,1 bis 1 000 g und vorzugsweise 1 g bis 50 g Lösemittel zugesetzt worden sind.

Ebenfalls von Vorteil kann es sein, wenn pro Gramm an eingesetzter Oxoverbindung eine solche Menge an eingeschlossener Enzym-haltiger Komponente zugesetzt wird, die eine Oxynitrilase-Aktivität zwischen 1 U und 10 000 U und bevorzugt 20 bis 2 000 U entwickelt.

Die Oxynitrilase, die auch als Mandelonitril-Lyase bezeichnet werden kann, wird bevorzugt als stereospezifische (R)-Oxynitrilase (zur Herstellung von (R)-Cyanhydrinen) oder (S)-Oxynitrilase (zur Herstellung von (S)-Cyanhydrinen) eingesetzt. Es ist aber auch möglich, Gemische von (R)- und (S)-Oxynitrilase in beliebigen Mischungsverhältnissen zu verwenden. Die Oxynitrilase wirkt als Katalysator für die stereospezifische Synthese von Cyanhydrinen aus den eingesetzten Oxoverbindungen und Cyaniddonoren.

Als besonders vorteilhaft haben sich Varianten des beanspruchten Verfahrens erwiesen, bei denen eine molekulargewichtsvergrößerte Oxynitrilase eingesetzt wird, deren Molmasse bevorzugt > 60 000 g/mol und insbesondere > 200 000 g/mol ist, und/oder eine Oxynitrilase, die sich in Zellen und hierbei vorzugsweise in gentechnisch veränderten Zellen, in Micellen oder in Mandelkernkleie befindet, wobei alle diese Enzym- haltigen Komponenten entsprechend der Erfindung natürlich noch von einem elastischen Gel eingeschlossen sein müssen.

Für das vorliegende Verfahren haben sich eine ganze Reihe an Möglichkeiten zur Molekulargewichtsvergrößerung als besonders geeignet gezeigt: Eine Möglichkeit der Molekulargewichtsvergrößerung besteht in der Quervernetzung der Oxynitrilase, was vorzugsweise mit Glutaraldehyd oder Diisocyanat erfolgt. Für eine weitere Art der Quervernetzung eignet sich erfindungsgemäß aber auch die alternative Umsetzung der Oxynitrilase mit Glutaraldehyd und/oder einem Diisocyanat und ggf. einem Trägermaterial, wie z. B. Amino- und/oder Hydroxygruppen-haltigen organischen oder anorganischen Materialien.

Insbesondere die kovalente Anbindung der Oxynitrilase an anorganische Trägermaterialien, wie vorzugsweise aktiviertes poröses Glas ist deshalb als Methode zur Molekulargewichtsvergrößerung besonders gut geeignet.

Darüber hinaus sieht die Erfindung auch die Molekulargewichtsvergrös-serung durch eine sogenannte Co-Quervernetzung der Oxynitrilase in Gegenwart von zwei Reagentien vor. Als ideal hat sich dabei die Coquervernetzung mit Glutaraldehyd, Chitosan, Polyethylenimin, Polyvinylamin oder Gelatine herausgestellt, wobei auch alle beliebigen Mischungen daraus in Frage kommen. Die Kombination von Glutaraldehyd mit Chitosan ist dabei besonders empfehlenswert.

Als weitere Verfahrensmöglichkeit zur Molekulargewichtsvergrößerung berücksichtigt die Erfindung eine Flocculation der Oxynitrilase, was vorzugsweise am besten mit Polyelektrolyten geschieht, wie z. B. Poly(dialkylammoniumchlorid), Chitosan, Diethylaminoethyldextran als typische Polykationen oder Polystyrolsulfonsäure, Cellulosesulfat, Sulfaethylcellulose, Dextransulfat, Polyacrylsäure und Copolymere als typische Polyanionen sowie beliebige Mischungen daraus.

Als Enzymquelle kann für die bevorzugten Verfahren zur Molekulargewichtsvergrößerung entweder das Enzym direkt verwendet werden, das damit in freier Form eingesetzt wird, oder entsprechende, die Oxynitrilase enthaltende Zellen; im letzteren Fall erfolgt die Quervernetzung der Oxynitrilase besonders effektiv direkt in einer Mandelkleie; erfindungsgemäß können aber auch gentechnisch veränderte Zellen und/oder künstliche Zellen, sog. Micellen, die solche Enzyme produzieren bzw. enthalten, verwendet werden.

Eine solche spezielle Molekulargewichtsvergrößerung kann notwendig werden, um den Anforderungen der nachfolgend erforderlichen Gelierungstechnologie gerecht zu werden. So wird zur Verhinderung der Permeabilität und damit eines "Leaching"-Verlusts von Enzymen im allgemeinen eine minimale Molmasse von 50000 g/mol benötigt. In diesem kritischen Bereich liegt beispielsweise auch die Molmasse der (R)-Oxynitrilase, so dass eine deutliche Erhöhung des Molekulargewichts Voraussetzung für eine effiziente Rückhaltung in der späteren Gel-Strukrur ist.

Anstelle der freien Oxynitrilasen können gemäß Erfindung für die Molekulargewichtsvergrößerung und anschließende Gelierung auch Zellen eingesetzt werden. Zum einen eignen sich dafür natürliche "Oxynitrilase-Quellen", vorzugsweise Mandelkernkleie. Daneben sieht das vorliegende Verfahren aber auch die Verwendung gentechnisch veränderter Zellen vor. Für die spätere Immobilisierung des Enzyms durch Gel-Einschluss und/oder Gel-Komplexierung eignen sich vorzugsweise Zellen mit einer erhöhten Enzymaktivität und solche, die ggf. zusätzlich eine erhöhte Zellwandpermeabilität aufweisen; aber auch Zellen mit einer Ein-Enzym-Aktivität sind geeignet. Die gewünschte Enzymaktivität wird bspw. durch Vernetzung bzw. eine Temperaturund/oder pH-Wert-Behandlung analog den bekannten Techniken erzielt. Die gewünschte Zellwandpermeabilität kann bspw. durch Behandlung mit organischen Lösemitteln sowie mit energetischen Methoden, wie z. B. Hochspannungsimpulsen, erreicht werden. Möglich ist aber auch der Einsatz von künstlichen Zellen, also von Micellen, was die Erfindung ebenfalls vorsieht.

Das für das vorliegende Verfahren als wesentlich zu bezeichnende Merkmal des Gel-Einschlusses und/oder der Gel-Komplexierung der vorher molekulargewichtsvergrößerten Oxynitrilase bzw. der eine solche Oxynitrilase enthaltenden Zellen, also der Enzym-haltigen Komponente, wird verwirklicht, indem die makroskopischen Eigenschaften der Gele, insbesondere deren Form betreffend, über eine Steuerung des Prozesses der Gelbildung gezielt beeinflusst werden. Als Methoden der Gelbildung werden dem Stand der Technik entsprechend und im Sinne der Erfindung vorzugsweise die Methoden zur Herstellung von linsenförmigen Gelen bzw. die Kryogelierung (vgl. z. B. DE-OS 198 27 552 und DE-PS 40 27 218) zur Herstellung von kugelförmigen Gelperlen angewendet.

Bei der Herstellung von linsenförmigen Gel-Enzym-Komponenten wird zur Gelierung die Lösung mit molekulargewichtsvergrößerter Oxynitrilase bzw. eine Lösung mit Zellen, die eine solche Oxynitrilase enthalten, mit einer Polymerlösung vermischt, was bevorzugt durch Umsetzung mit Polyvinylakohol und unter Ausbildung entsprechender Polyvinylalkohol(PVA)-Gele erfolgt, und das Gemisch anschließend tropfenweise aufgetragen, wobei unter Entweichen von Wasser der Gelierungs-Prozess einsetzt. Durch spontane Gelierung werden dann linsenförmige Gele erhalten. Dieser Prozess der Gelbildung ist u.a. beschrieben in der deutschen Offenlegungsschrift 198 27 552.

Alternativ hierzu kann aber auch die Umsetzung der molekulargewichtsvergrößerten Oxynitrilase bzw. der Oxynitrilase-haltigen Zellen mit einer Polycarbamoylsulfonatquelle durchgeführt werden, wobei sich entsprechende Polycarbamoylsulfonat-Gele ausbilden. Ein Vorteil dieser Vorgehensweise liegt in der kovalenten Einbindung der Enzyme in das entstehende Netzwerk während des Gelierungsprozesses. Neben einem Gelierungsvorgang findet somit simultan auch eine Molmassenvergrößerung der einzuschließenden Enzym-Systeme statt.

Als dritte Variante der gemäß Erfindung getrennten Gelbildung ist eine sogenannte Kryogelierung vorgesehen, bei der die Gelierung durch das Eintropfen der mit einer Polymer-Lösung vermischten Oxynitrilase-Lösung bzw. einer Lösung mit Oxynitrilase-haltigen Zellen in eine tiefkalte Flüssigkeit erfolgt. Die Flüssigkeit darf dabei allerdings nicht mit der Gel-haltigen Lösung mischbar sein. Vorzugsweise werden deshalb flüssiger Stickstoff oder andere geeignete kondensierbare Gase verwendet. Nach anschließendem gezieltem Auftauen werden die enzymhaltigen Gele in Form von kugelförmigen "Gelperlen" erhalten, die einen Durchmesser von 50 bis 5000 µm besitzen.

In der Praxis hat es sich als vorteilhaft erwiesen, wenn für das Verfahren gemäß Erfindung linsenförmige enzymhaltige gelartige Einschlüsse oder Komplexe mit einem Durchmesser von mindestens ca. 0,5 mm bis 10 mm, vorzugsweise 3 mm bis 10 mm, sowie mit einer Dicke von 20 µm bis 5000 µm, vorzugsweise 200 µm bis 2000 µm eingesetzt werden. Ebenfalls bestens geeignet sind aber auch kugelförmige enzymhaltige Gele, vorzugsweise mit einem Durchmesser von 50 bis 5000 µm und besonders bevorzugt von 200 bis 2000 µm. Diese hervorragende Eignung der in einem Gel eingeschlossenen und/oder komplexierten Enzym-haltigen Komponenten für technische Prozesse beruht zum einen auf deren exzellenten Diffusionseigenschaften, gewährleistet durch die geringe Dicke der linsenförmigen enzymhaltigen Gele, sowie auf der hervorragenden Abtrennbarkeit dieser Linsen, insbesondere durch Filtration, was durch deren Form und Größe bedingt ist. Zudem besitzen sowohl die Linsen wie auch die sog. Gelperlen eine außergewöhnlich hohe Elastizität, einhergehend mit einer hohen Abriebfestigkeit und einem vernachlässigbaren Abriebsverlust. Die linsen- bzw. perlenförmigen enzymhaltigen Gele sind somit auch in stark gerührten bzw. durch Scherkräfte stark beanspruchten Reaktorsystemen hervorragend einsetzbar.

Zusammenfassend ist festzuhalten, dass zur Durchführung des erfindungsgemäßen Verfahrens bevorzugt die von einem Gel eingeschlossene und/oder komplexierte Enzym-haltige Komponente zunächst in das vorgelegte Reaktionssystem gegeben wird. Danach erfolgt die Zugabe der weiteren Reaktionskomponenten, wobei insgesamt die Zugabereihenfolge der einzelnen Komponenten frei gewählt werden kann. Die abschließende Aufarbeitung erfolgt beispielsweise durch eine einfache Abtrennung der makroskopischen Gel-Enzympartikel von der Reaktionsmischung durch Filtration, wobei die abfiltrierten, eingeschlossenen und/oder komplexierten Enzympartikel anschließend sofort und in der Regel ohne weitere Aufarbeitung wiederverwendet werden können. Das erhaltene Filtrat mit der erhaltenen Reaktionsmischung wird nach den bekannten Verfahren weiter aufgearbeitet. Alternativ kann aber bei Reaktionen im 2-Phasensystem auch nur die organische Phase abgetrennt und weiterverarbeitet werden, während die "makroskopischen Enzympartikel" in der wässrigen Phase verbleiben und dieses System für die nächste Reaktion wiederverwendet wird. Auch die evtl. nicht umgesetzte Oxoverbindung kann selbstverständlich rezykliert werden. Die Wiedereinsetzbarkeit der enzymhaltigen Gellinsen bzw. Gelperlen erstreckt sich über einen mindestens mehrwöchigen, z. T. aber auch mehrmonatigen Produktionszeitraum, wobei keinerlei Abnahme der katalytischen Aktivität der von einem gelartigen Medium eingeschlossenen/komplexierten Oxynitrilasen festzustellen ist. Herauszuheben sind vor allem das hohe Maß an Aktivität und Stabilität der Oxynitrilasen, der effiziente Reaktionsverlauf bei dem erfindungsgemäßen Verfahren mit Hilfe solcher enzymhaltigen-Gelsysteme, sowie die hohe molekulare und makroskopische Stabilität der Enzym-haltigen Komponenten.

Diese Vorteile der zum einen rührmechanisch stabilen und zum anderen gleichzeitig langzeitaktiven und zu hohen Ausbeuten und Enantioselektivitäten führenden Enzym-Einschluss-Verbindungen werden durch die voneinander getrennte Abfolge von Molekulargewichtsvergrößerung und die sich anschließende Gel-Einschluss- oder Komplex-Bildung erreicht.

Die gewünschten Cyanhydrine werden mit dem erfindungsgemäßen Verfahren in hohen Ausbeuten > 80 % und guten bis hervorragenden Enantioselektivitäten bis > 99 % erhalten. Die Kapseln weisen im übrigen eine Rezyklingrate von mindestens 20 Zyklen auf, ohne dass dabei ein Aktivitäts-, Ausbeute- und insbesondere Enantioselektivitäts-Verlust auftritt.

Beschrieben wird ein Verfahren zur Herstellung von optisch aktiven Cyanhydrinen durch Umsetzung von Oxoverbindungen, wie bspw. Aldehyde und/oder Ketone, mit Blausäure und/oder mit einem Cyaniddonor in Gegenwart einer Einschlusssverbindung, die eine molekuargewichtsvergrößerte Oxynitrilase enthält, bei dem man eine Oxynitrilase verwendet, die im wesentlichen in zwei voneinander unabhängigen Schritten erstens durch Quervernetzung molekuralgewichtsvergrößert und anschließend in eine Gel-Einschluss- und/oder eine Gel-Komplex-Verbindung überführt worden ist. Das Verfahren, das insbesondere bei Temperaturen zwischen - 10 und+ 30 °C sowie bei pH-Werten von 3,0 bis 6,0, vorzugsweise in einem wässrigen oder wasserhaltigen System, aber auch in organischen Lösemitteln durchgeführt wird, bedient sich einer Enzym-haltige Komponente, bei der das Enzym selbst insbesondere durch eine Co-Quervernetzung oder durch Flocculation molekulargewichtsvergrößert ist und entweder in freier Form oder in natürlichen oder künstlichen Zellen eingeschlossen angewendet wird. Entscheidend bei diesem Verfahren ist die Tatsache, dass das Enzym in ein Gel eingeschlossen bzw. komplexiert ist, wodurch bei nahezu unverändert hoher Enzymaktivität vernachlässigbar kleine Abriebs- und Leaching-Verluste auftreten, was das Verfahren äußerst effektiv und wirtschaftlich macht.

Die nachfolgenden Beispiele und die beigefügte Figur zeigen die Vorteile des vorliegenden erfindungsgemäßen Verfahrens zur Herstellung von optisch aktiven Cyanhydrinen.

### Beispiele

### Beispiel 1 (Vergleichsbeispiel):

### Reaktion mit freiem Enzym bei pH 5.5

Zu einem Lösemittelgemisch, bestehend aus 1.6 mL Methyl-tert.-butylether und 2.4 mL n-Hexan wurden 106 mg frisch destillierter Benzyaldehyd gegeben.
Anschließend wurden zu diesem Gemisch nacheinander 4 mL eines 50 mM Citratpuffers mit pH 5.5, 0.15 mL einer (R)-Oxynitrilaselösung (15U; isoliert, aber nicht aufgereinigte (R)-Oxynitrilase; Hersteller: ASA Spezialenzyme GmbH, Braunschweig) und 473 mg einer 20%-igen Blausäurelösung (3.5 Äquivalente) bei Raumtemperatur hinzugefügt. Das Reaktionsgemisch wurde dann 2 Stunden bei Raumtemperatur gerührt und anschließend mit 10 mL Methyl-tert.-butylether (MTBE) und 10 mL Citratpuffer versetzt. Die organische Phase wurde abgetrennt und die wässrige Phase mit 4 x 15 mL MTBE gewaschen. Schließlich wurden die vereinigten organischen Phasen mit Natriumsulfat getrocknet und nach Filtration im Vakuum (bis 20 mbar) von den flüchtigen Bestandteilen befreit. Das gewünschte Produkt (R)-Mandelonitril fiel in einer Ausbeute von 95% und mit einer Enantioselektivität von 77% ee an.

### Beispiel 2 (Vergleichsbeispiel):

### Reaktion mit freiem Enzym bei pH 4.5

Zu einem Lösemittelgemisch, bestehend aus 1.6 mL Methyl-tert.-butylether und 2.4 mL n-Hexan wurden 106 mg frisch destillierter Benzyaldehyd gegeben. Anschließend fügte man zu diesem Gemisch nacheinander 4 mL eines 50 mM Citratpuffers mit pH 4.5, 0.15 mL einer (R)-Oxynitrilaselösung (15U; isoliert, aber nicht aufgereinigte (R)-Oxynitrilase; Hersteller: ASA Spezialenzyme GmbH, Braunschweig) und 473 mg einer 20%-igen Blausäurelösung (3.5 Äquivalente) bei Raumtemperatur hinzu. Das Reaktionsgemisch wurde dann 2 Stunden bei Raumtemperatur gerührt und anschließend mit 10 mL MTBE und 10 mL Citratpuffer versetzt. Die organische Phase wurde abgetrennt und die wässrige Phase mit 4 x 15 mL Methyl-tert.-Butylether (MTBE) gewaschen. Schließlich wurden die vereinigten organischen Phasen mit Natriumsulfat getrocknet und nach Filtration im Vakuum (bis 20 mbar) von den flüchtigen Bestandteilen befreit. Das gewünschte Produkt (R)-Mandelonitril fiel in einer Ausbeute von 85% und mit einer Enantioselektivität von 95% ee an.

### Beispiel 3:

### Herstellung der PVAL-Gel-immobilisierten (R)-Oxynitrilasen

Im ersten Schritt wurde eine Coquervernetzung der (R)-Oxynitrilase mit Chitosan und Glutardialdehyd (GDA)durchgeführt: Dazu wurden unter Rühren zunächst 1.5 g Chitosan in 98.5 g einer 0.5%-igen Essigsäure gelöst und der pH-Wert dieser Chitosanlösung mit 1 M Natronlauge auf pH 5.5 eingestellt. Anschließend wurden zu 4 g dieser Chitosanlösung (entsprechend 60 mg Chitosan) langsam 7.89 g Enzymlösung (60 mg Protein) zugetropft. Danach wurde dieses Gemisch langsam mit 200 µL einer 50%-igen Glutardialdehydlösung (pH 5.5) versetzt, worauf die entstandene Mischung unter Rühren bei 4 °C im Laufe von 16 Stunden vernetzte. Die coquervernetzte (R)-Oxynitrilase wurde anschließend in einem zweiten Schritt in Polyvinylakohol unter Ausbildung der PVAL-gelimmobilisierten (R)-Oxynitrilase eingeschlossen: Dazu löste man 6 g Polyethylenglykol (PEG 1000) in 74 g Wasser und fügte langsam 10 g Polyvinylalkohol (PVA 17-99) hinzu. Die Mischung wurde in der Mikrowelle langsam erhitzt (T>90°C) und nach Abkühlen mit 2.07 g des Chitosan/Glutaraldehyd-coquervernetzten, abzentrifugierten Enzyms und 7,9 g Wasser versetzt. Nach Dispergieren mit dem Magnetrührer erfolgte die Herstellung der Linsen durch Auftropfen der Polymersuspension mit quervernetztem Enzym auf eine Petrischale mit Hilfe des LentiKat®-Printers. Die Linsen wurden auf < 50 % der Ausgangsmasse getrocknet. Anschließend erfolgte das Rückquellen der Linsen in einer Natriumsulfat-Lösung der Konzentration 100 mmol/L. Die PVAL-gelimmobilisierten (R)-Oxynitrilasen wurden so als hochelastische Linsen mit einem definierten Durchmesser von 5 mm erhalten. Die Aktivität lag bei 8,16 U pro g Feuchtmasse.

### Beispiel 4:

### Herstellung der PVAL-Gel-immobilisierten (R)-Oxynitrilasen

Im ersten Schritt wurde eine Coquervernetzung der (R)-Oxynitrilase mit Chitosan und Glutardialdehyd (GDA) durchgeführt. Dazu wurden unter Rühren zunächst 1,5 g Chitosan in 98,5 g einer 0,5%-igen Essigsäure gelöst und der pH-Wert dieser Chitosanlösung mit 1 M Natronlauge auf pH 5.5 eingestellt. Anschließend wurden zu 20 g dieser Chitosanlösung (entsprechend 300 mg Chitosan) langsam 39.5 mL Enzymlösung (300 mg Protein) zugetropft. Danach wurde dieses Gemisch langsam mit 1500 µL einer 50%-igen Glutardialdehydlösung (pH 5.5) versetzt, worauf die entstandene Mischung dann unter Rühren bei 4 °C im Laufe von 16 Stunden vernetzte. Die coquervernetzte (R)-Oxynitrilase wurde dann in einem zweiten Schritt in Polyvinylakohol unter Ausbildung der PVAL-gelimmobilisierten (R)-Oxynitrilase eingeschlossen: Dazu löste man 6 g Polyethylenglykol (PEG 1000) in 74 g Wasser und fügte langsam 10 g Polyvinylalkohol (PVA 17-99) hinzu. Die Mischung wurde in der Mikrowelle langsam erhitzt (T>90°C) und nach Abkühlen mit 8.42 g des Chitosan/Glutaraldehyd-coquervernetzten, abzentrifugierten Enzyms (300 mg Protein) und 1.58 g Wasser versetzt. Nach Dispergieren mit dem Magnetrührer erfolgte die Herstellung der Linsen durch Auftropfen der Polymersuspension mit quervernetztem Enzym auf eine Petrischale mit Hilfe des LentiKat®-Printers. Die Linsen wurden auf < 50 % der Ausgangsmasse getrocknet. Anschließend erfolgte das Rückquellen der Linsen in einer Natriumsulfat-Lösung der Konzentration 100 mmol/L. Die PVAL-gelimmobilisierten (R)-Oxynitrilasen wurden so als hochelastische Linsen mit einem definierten Durchmesser von 5 mm erhalten. Die Aktivität lag bei 40 U pro g Feuchtmasse.

### Beispiel 5:

### Reaktion mit PVAL-Gel-immobilisierter (R)-Oxynitrilase

In 4 mL eines 50 mM Citratpuffers (mit pH 4.5) wurden 1.84 g der Kapseln mit PVAL-gelimmobilisierter (R)-Oxynitrilase (8.16 U/g; hergestellt gemäß Beispiel 3) vorgelegt und dazu ein Lösemittelgemisch, bestehend aus 1.6 mL Methyl-tert.-butylether und 2.4 mL n-Hexan, sowie 106 mg frisch destillierter Benzyaldehyd gegeben. Anschließend fügte man 473 mg einer 20%igen Blausäurelösung (3.5 Äquivalente) bei Raumtemperatur hinzu. Das Reaktionsgemisch wurde dann 2 Stunden bei Raumtemperatur gerührt und anschließend mit 10 mL MTBE versetzt. Daraufhin wurde die organische Phase abgetrennt, die wässrige Phase mit 2 x 15 mL MTBE gewaschen und die vereinigten organischen Phasen wurden mit Natriumsulfat getrocknet und nach Filtration im Vakuum (bis 20 mbar) von den flüchtigen Bestandteilen befreit. Das gewünschte Produkt (R)-Mandelonitril fiel so in einer Ausbeute von 93 % und mit einer Enantioselektivität von 94 % ee an.

### Beispiel 6:

### Recyclingreihe (insgesamt 25 Versuche) der Cyanhydrinsynthese mit PVAL-Gel-immobilisierter (R)-Oxynitrilase

Im einleitenden Versuch 1 dieser Recyclingreihe wurden in 12 mL eines 50 mM Citratpuffers (mit pH 4,5) 11,0 g der Kapseln mit PVAL-gelimmobilisierter (R)-Oxynitrilase (40 U/g; hergestellt gemäß Beispiel 4) vorgelegt und dazu ein Lösemittelgemisch bestehend aus 4,8 mL Methyl-tert.butylether und 7,2 mL n-Hexan, sowie 318 mg frisch destillierter Benzyaldehyd gegeben. Anschließend fügte man 1,4 g einer 20%-igen Blausäurelösung (3,5 Äquivalente) bei Raumtemperatur hinzu. Das Reaktionsgemisch wurde dann 2 Stunden bei Raumtemperatur gerührt und anschließend mit 10 mL MTBE versetzt. Daraufhin wurde die organische Phase abgetrennt und die wässrige Phase mit 2 x 15 mL MTBE gewaschen, die vereinigten organischen Phasen wurden mit Natriumsulfat getrocknet und nach Filtration im Vakuum (bis 20 mbar) von den flüchtigen Bestandteilen befreit. Das gewünschte Produkt (R)-Mandelonitril fiel in einer Ausbeute von 74% und mit einer Enantioselektivität von 91% ee an. Anschließend wurden für den 2. bis 21. Versuch jeweils die in der vorhergehenden Reaktion verbliebenen Pufferlösungen enthaltend die Kapseln mit PVAL-gelimmobilisierter (R)-Oxynitrilase (40 U/g; hergestellt gemäß Beispiel 4) wiederverwendet, indem man erneut ein Lösemittelgemisch, bestehend aus 4.8 mL Methyl-tert.-butylether und 7.2 mL n-Hexan, sowie 318 mg frisch destillierter Benzyaldehyd hinzufügte. Anschließend gab man 1.4 g einer 20%igen Blausäurelösung (3.5 Äquivalente) bei Raumtemperatur hinzu, rührte das Reaktionsgemisch dann 2 Stunden bei Raumtemperatur und versetzte anschließend mit 10 mL MTBE. Die organische Phase wurde abgetrennt, die wässrige Phase mit 2 x 15 mL MTBE gewaschen und die vereinigten organischen Phasen wurden mit Natriumsulfat getrocknet und nach Filtration im Vakuum (bis 20 mbar) von den flüchtigen Bestandteilen befreit. Das gewünschte Produkt (R)-Mandelonitril fiel in den in der Tabelle angegebenen Ausbeuten (%) und mit der jeweiligen Enantioselektivität ee (%) an. Die verbliebene Pufferlösung mit den Kapseln mit PVAL-gelimmobilisierter (R)-Oxynitrilase (40 U/g; hergestellt gemäß Beispiel 4) wurde jeweils für den nächsten Recyclingversuch wiederverwendet.

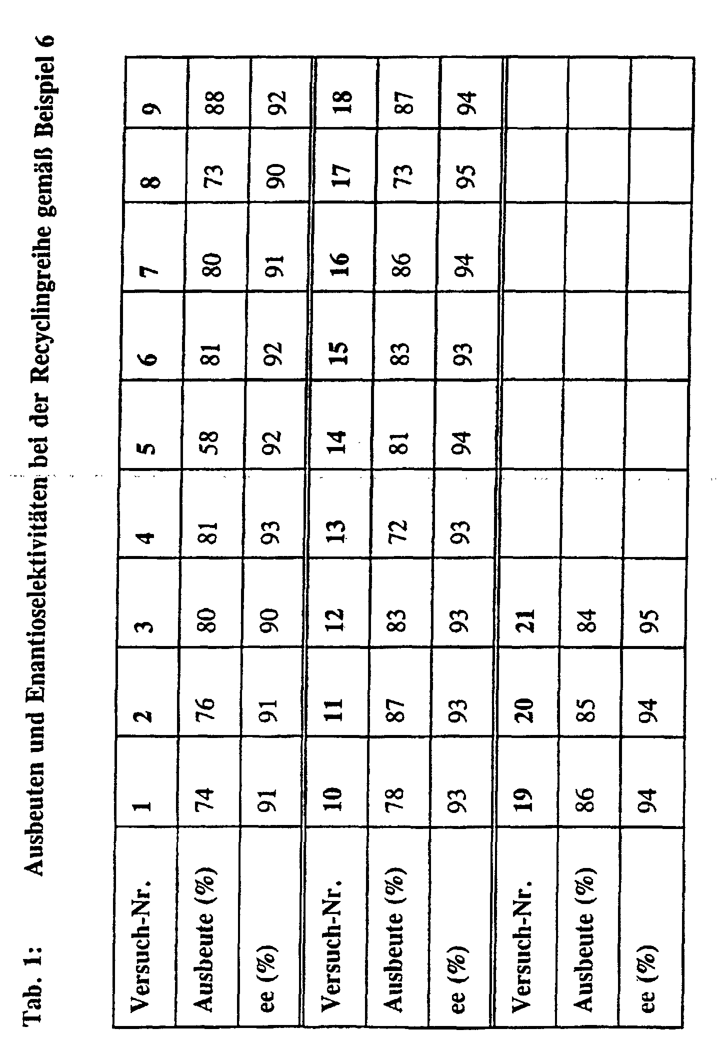

### Beispiel 7:

### Versuche zur Rückhaltung der (R)-Oxynitrilase bei Verwendung der PVAL-Gel-immobilisierten (R)-Oxynitrilase (in Form von Kapseln, sog. Lenti-Kats) bei der Spaltungsreaktion von Mandelonitril:

Es wurden 0.6 g der Kapsel mit PVAL-gelimmobilisierter (R)-Oxynitrilase (8,16 U/g; hergestellt nach Beispiel 3) in 100 mL eines 50 mM Citratpuffers gegeben und 143 Stunden bei Raumtemperatur gerührt. Danach wurden die Kapseln von der Lösung abgetrennt. Sowohl die Kapseln als auch der Überstand wurden bezüglich ihrer Enzym-Aktivität getestet. Im Vergleich dazu wurde in einem anderen Versuch das immobilisierte Enzym direkt nach der Herstellung auf die Aktivität hin getestet.

Der Aktivitätstest wurde wie folgt durchgeführt (am Beispiel der Kapseln): Zu 100 mL einer 1 mmol/L Mandelonitrillösung in 50 mM Citratpuffer wurden 0,6 g des immobilisierten Enzyms (Kapseln) gegeben. Der Versuch wurde bei einem pH-Wert von 3.75 und einer Temperatur von 20 °C durchgeführt. Der Reaktionsverlauf wurde mit einem Photometer verfolgt. Die Spaltung von Mandelonitril lässt sich bei 250 nm photometrisch über die Zunahme der Benzaldehydkonzentration (Extinktion) verfolgen.

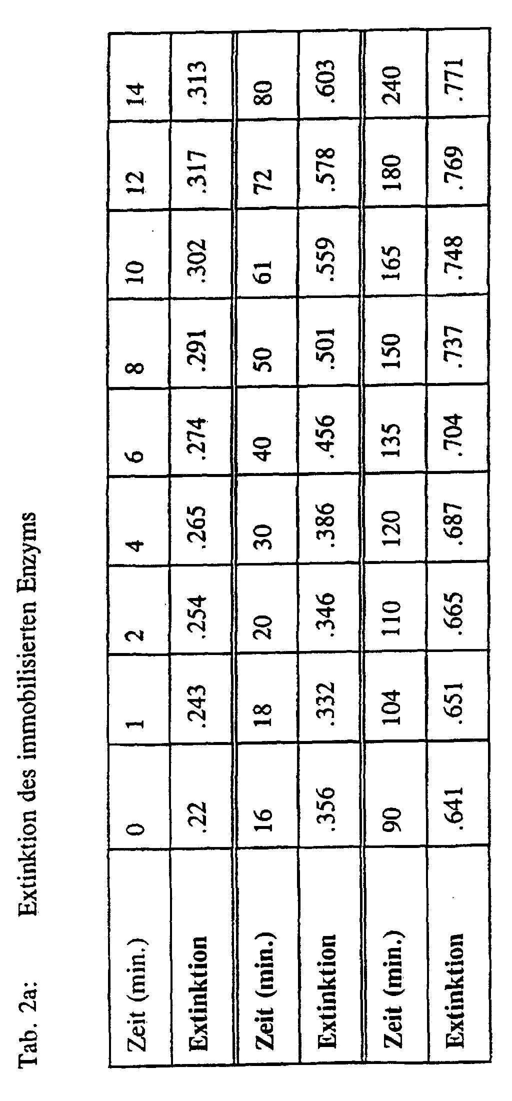

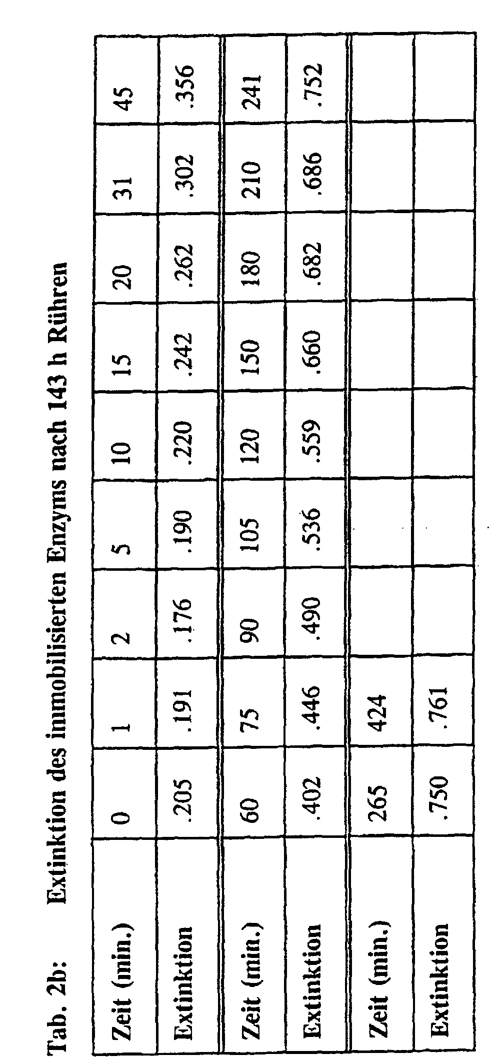

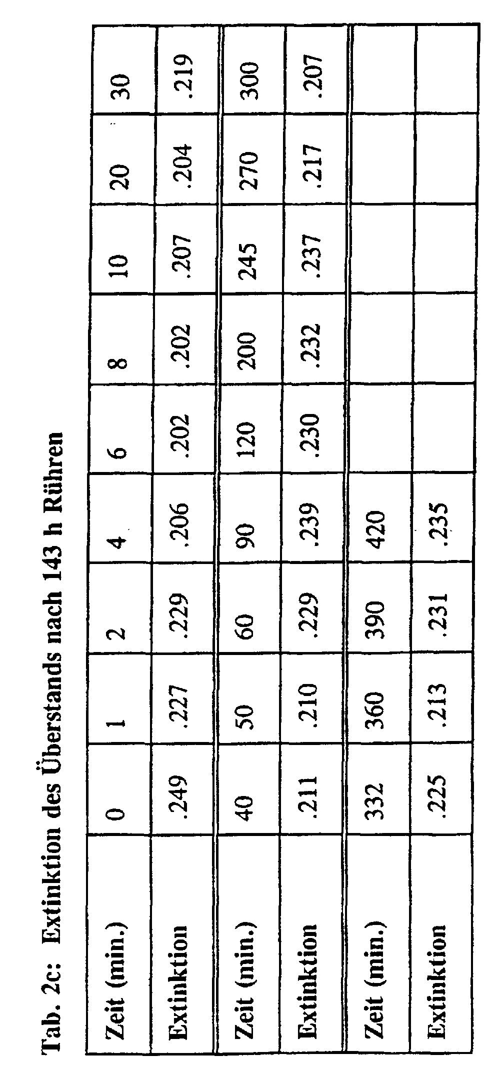

### Beispiel 8:

### Recyclingreihe (insgesamt 7 Versuche) der Cyanhydrinsynthese mit PVAL-Gel-immobilisierter (R)-Oxynitrilase

Im einleitenden Versuch 1 dieser Recyclingreihe wurden in 6 mL eines 50 mM Citratpuffers (mit pH 4.5) 3,70 g der Kapseln mit PVAL-gelimmobilisierter (R)-Oxynitrilase (40 U/g; hergestellt gemäß Beispiel 4) vorgelegt und dazu 6 mL Diisopropylether sowie 159 mg frisch destillierten Benzyaldehyds gegeben. Anschließend fügte man bei Raumtemperatur 710 mg einer 20%-igen Blausäurelösung (3.5 Äquivalente) hinzu. Das Reaktionsgemisch wurde dann 3 Stunden bei Raumtemperatur gerührt und anschließend mit 10 mL MTBE versetzt. Anschließend wurde die organische Phase abgetrennt und die wässrige Phase mit 2 x 10 mL MTBE gewaschen. Die vereinigten organischen Phasen wurden mit Natriumsulfat getrocknet und nach Filtration im Vakuum (bis 20 mbar) von den flüchtigen Bestandteilen befreit. Das gewünschte Produkt (R)-Mandelonitril fiel so in einer Ausbeute von 84% und mit einer Enantioselektivität von 99,8 % ee an.

Dann wurden für den 2. bis 7. Versuch die in der jeweils vorhergehenden Reaktion verbliebenen Kapseln mit PVAL-gelimmobilisierter (R)-Oxynitrilase (40 U/g; hergestellt gemäß Beispiel 4) wiederverwendet, indem man dazu erneut 6 mL eines 50 mM Citratpuffers (mit pH 4.5), 6 mL Düsopropylether sowie 159 mg frisch destillierten Benzyaldehyds gab. Anschließend wurden bei Raumtemperatur 710 mg einer 20%-igen Blausäurelösung (3.5 Äquivalente) hinzugefügt. Nach 3 stündigem Rühren bei Raumtemperatur wurde das Reaktionsgemisch mit 10 mL MTBE versetzt, die organische Phase wurde abgetrennt und die wässrige Phase mit 2 x 10 mL MTBE gewaschen. Die vereinigten organischen Phasen wurden mit Natriumsulfat getrocknet und nach Filtration im Vakuum (bis 20 mbar) von den flüchtigen Bestandteilen befreit. Das gewünschte Produkt (R)-Mandelonitril fiel in den in der Tabelle 3 angegebenen Ausbeuten (%) und mit den ebenfalls darin enthaltenen Enantioselektivität ee (%) an. Die verbliebenen Kapseln mit PVAL-gelimmobilisierter (R)-Oxynitrilase (40 U/g; hergestellt gemäß Beispiel 4) wurden jeweils für den nächsten Recyclingversuch wiederverwendet.

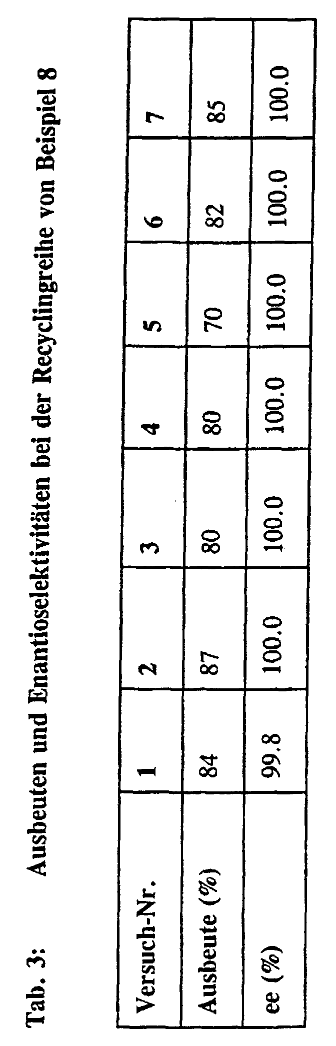

### Zusammenfassung der Beispiele:

Die Versuche zur Cyanhydrinsynthese fanden unter Verwendung einer nichtaufgereinigten (R)-Oxynitrilase statt. Dieses Enzym ist zwar von minderer Reinheit und Aktivität verglichen mit den hochaufgereinigten Spezialenzymen, bietet dafür aber ökologische und ökonomische Vorteile aufgrund des erheblich geringeren Preises.

Die Anwendung dieses Enzyms (Hersteller: ASA Spezialenzyme GmbH, Braunschweig) in der freien Form gemäss den in der Literatur beschriebenen Versuchsbedingungen für die im Zweiphasensystem durchgeführte Cyanhydrin-Synthese ergab bei pH 5,5 einen ee-Wert von 77 % ee bei einer Ausbeute von 95 %, wenn eine Enzymaktivität von 15 U/mmol eingesetzt wurde (Vergleichsbeispiel Beispiel 1). Eine Verbesserung der Enantioselektivität konnte bei der Durchführung des Versuchs mit diesem freien ASA-Enzym bei pH 4,5 erzielt werden. Hierbei wurde eine Ausbeute von 85 % bei hohen 95 % ee für die Enantioselektivität erzielt (Vergleichsbeispiel Beispiel 2).

Das Vergleichsbeispiel (Beispiel 2) wurde aufgrund des deutlich höheren ee-Werts somit als Vergleichsversuch für die Experimente mit den verkapselten Enzymen ausgewählt. Die Versuche mit den verkapselten Enzymen wurden ebenfalls bei pH 4,5 durchgeführt, da eine hohe Enantioselektivität >90 % ee vorrangiges Ziel der enzymatischen Cyanhydrin-Synthese ist.

Der zu Beispiel 2 analoge Versuch mit der Polyvinylalkohol(PVAL)-immobilisierten Oxynitrilase ergab dann trotz Molekulargewichtsvergrößerung und Verkapselung ein vergleichbares gutes Ergebnis sowohl bezüglich Ausbeute als auch Enantioselektivität. Bei Einsatz von (R)-Oxynitrilase-haltigen Enzymkapseln mit einer Aktivität von 15 U/mmol erfolgte die Herstellung des gewünschten Mandelonitrils mit vergleichbaren 94 % ee und 93 % Ausbeute (Beispiel 5). Sowohl Enzymaktivität als auch die übrigen Reaktionsbedingungen (Temperatur, Solvens, Reaktionszeit, HCN-Äquivalente) sind dabei exakt diesselben wie beim Vergleichsversuch Beispiel 2. Die in diesem Versuch (Beispiel 5) verwendeten Kapseln mit einer Aktivität von 8,16 U/g wurden gemäß der in Beispiel 3 beschriebenen Vorschrift hergestellt.

Die Herstellung der Kapseln erfolgt unter definierten Bedingungen. Repräsentative Versuchsvorschriften sind in den Versuchsvorschriften in den Beispiel 3 und 4 (jeweils für (R)-Oxynitrilasen) beschrieben. Insgesamt können zur Herstellung der Kapseln die Reaktionsbedingungen durchaus variiert werden und Kapseln mit den unterschiedlichsten Aktivitäten pro g Kapseln (Feuchtmasse) hergestellt werden. Der mit Kapseln der Beladung 8,16 U/g durchgeführte Versuch (Beispiel 5) wurde bereits beschrieben:
93 % Ausbeute; 94 % ee. Mit der Beladung von 40 U/g hergestellte Kapseln ergaben beispielsweise bei Verwendung in der asymmetrischen Cyanhydrin-Synthese das Mandelonitril-Produkt mit einer Ausbeute von 81 % und 89 % ee (Beispiel 6, Versuch 1 der Recyclingreihe).

Basierend auf diesem Versuch, Versuch 1 der Recyclingreihe (Beispiel 6) wurde dann eine insgesamt 21-Zyklen umfassende Reihe untersucht, anhand deren Ergebnisse das hohe Potential einer Wiederverwendbarkeit der Enzymkapseln aufgezeigt werden kann (Beispiel 6). Demnach bleibt bei stetigem Recyceln der Kapseln die Ausbeute nahezu konstant im Rahmen des Ausgangswerts, auch nach 20-maligem Recyceln! Bzgl. der Enantioselektivität ist überraschenderweise keinerlei Abfall zu erkennen. Vielmehr bleibt die Enantioselektivität ee konstant bzw. steigt im Laufe des Wiedereinsetzens noch weiter leicht an, von anfangs 91 % ee auf bis zu 95 % ee nach 20-maligem Wiedereinsatz, und könnte auf eine zunehmende Stabilisierung des Enzyms in der "Matrix" innerhalb der Kapseln zurückzuführen sein. Darüber hinaus haben sich die Kapseln als langzeitstabil erwiesen und verändern sich auch nicht hinsichtlich Flexibilität, Größe und Elastizität.

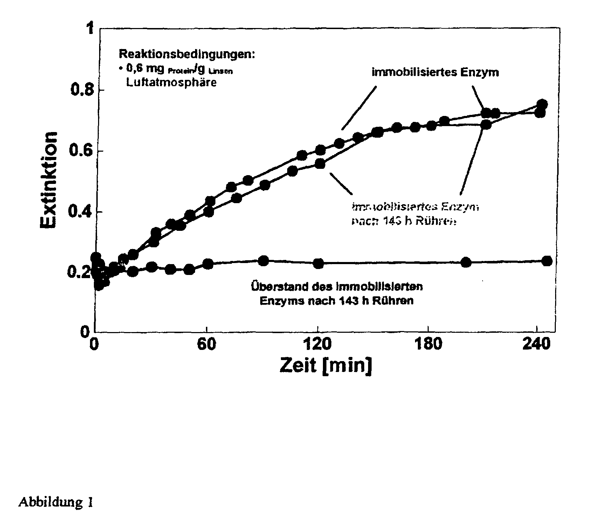

Das hohe Recyclingpotential wird auch durch die Untersuchungen zur Rückhaltung der in den Kapseln gebundenen (R)-Oxynitrilase bei Verwendung der PVAL-Gel-immobilisierten (R)-Oxynitrilase (in Form von Lenti-Kats) unterstrichen (Beispiel 7). Diese Versuche zur "Leachingrate" - also des Katalysatorverlusts während der Reaktion bzw. wiederholtem Einsatz - wurden anhand der Spaltungsreaktion von Madelonitril durchgeführt (da anhand dieser Reaktion die Enzymaktivität i.a. bestimmt wird). Zum Einsatz kamen die nach Beispiel 4 hergestellten Kapseln. Die Ergebnisse sind tabellarisch in Tabelle 2 und graphisch in Abbildung 1 aufgeführt. Demnach konnte keinerlei "Leaching", also kein Verlust an Katalysatoraktivität während des Reaktionszeitraums beobachtet werden. So bleibt die Extinktion für den Überstand des immobilisierten Enzyms nach 143-stündigem Rühren konstant, einhergehend mit keinerlei zusätzlicher Enzymaktivität des Enzyms im Überstand infolge eines theoretisch möglichen Austritts des Enzyms aus den Kapseln in die freie Lösung. Der nahezu identische Verlauf der Enzymaktivitäten der frisch hergestellten Enzymkapseln mit denen nach 143-stündigem Rühren zeigt, dass auch nach 143-stündigem Rühren keine Änderung der Enzymaktivität erfolgt. Somit tritt weder ein "Katalysatorleaching" auf, noch tritt ein Aktivitätsverlust des gebundenen Katalysators in einem langen Beobachtungszeitraum von 143 Stunden ein.

In einem weiteren Recyclingversuch konnte zudem gezeigt werden, dass selbst mit Kapseln, die ein nicht-aufgereinigtes Enzym enthalten, hervorragende Enantioselektivitäten von > 99 % ee erzielt werden können (Beispiel 8, Tabelle 3). Somit führt die Verkapselung der Enzyme nicht nur zu einer hohen Langzeitstabilität (oftmalige Einsetzbarkeit), sondern stabilisiert auch das Enzym durch die verkapselte Form, so dass hohe ee-Werte von >99 % ee erzielt werden können. Für die Versuche dieser Recyclingreihe wurden eine erhöhte Anzahl an Enzymkapseln mit einer Gesamtaktivität von ca. 100 U/mmol eingesetzt; zum Einsatz kamen die gemäß Beispiel 4 hergestellten Enzymkapseln. Zudem erfolgte die Anwendung optimierter Reaktionsbedingungen, so u.a. die Verwendung des Lösemittels Diisopropylether und eine längere Reaktionszeit. Bereits im einleitenden Versuch 1 wurde unter diesen Reaktionsbedingungen für das (R)-Mandelonitril eine Ausbeute von 84 % bei einer Enantioselektivität von 99,8 % ee erzielt. In den folgenden Recyclingzyklen bestätigte sich erneut der bei der bereits in Beispiel 7 beschriebenen Recyclingreihe gezeigte Trend: Zum einen steigt der bereits sehr hohe ee-Wert schon ab der zweiten Versuchsreihe weiter an auf die maximal mögliche Enantioselektivität von 100 % ee (siehe auch Tabelle 3), wobei der ee-Wert auch bei den restlichen 5 Recyclingreihen bei diesen 100 % ee bleibt, zum anderen bleiben die Ausbeuten im relativ konstanten Bereich von 70 bis 87 %, einhergehend mit einer durchschnittlichen Gesamtausbeute von > 80 %. Insbesondere durch die hohe Enantioselektivität von 99,8 bis 100 % ee zeigt sich ein hohes Anwendungspotential der enzymkatalysierten Cyanhydrin-Synthese unter Verwendung von (R)-Oxynitrilase-haltigen Enzymkapseln gemäß vorliegender Erfindung.

## Patentansprüche

1. Verfahren zur Herstellung von optisch aktiven Cyanhydrinen durch Umsetzung von Oxoverbindungen mit Blausäure und/oder mit einem Cyaniddonor und in Gegenwart einer Einschlussverbindung, die eine molekulargewichtsvergrößerte Oxynitrilase enthält, **dadurch gekennzeichnet, dass** man eine Oxynitrilase verwendet, die im wesentlichen in zwei voneinander unabhängigen Schritten erstens durch Quervernetzung molekulargewichtsvergrößert und anschließend in eine Gel-Einschluss- und/oder eine Gel-Komplex-Verbindung überführt worden ist.

2. Verfahren nach Anspruch 1, **dadurch gekennzeichnet, dass** die Herstellung bei Reaktionstemperaturen von - 10 bis + 30 °C, insbesondere von 5 bis 25 °C, erfolgt.

3. Verfahren nach einem der Ansprüche 1 oder 2, **dadurch gekennzeichnet, dass** man die Herstellung bei pH-Werten von 3,0 bis 6,0 durchführt.

4. Verfahren nach einem der Ansprüche 1 bis 3, **dadurch gekennzeichnet, dass** die Umsetzung in einem wässrigen oder wasserhaltigen System, vorzugsweise in einem System der Reihe Wasser, Wasser/Ethanol, Wasser/Methanol, Wasser/Essigsäureethylester, Wasser/Diisopropylether, Wasser/Methyl-tert-butylether und Wasser/Hexan erfolgt.

5. Verfahren nach einem der Ansprüche 1 bis 4, **dadurch gekennzeichnet, dass** die Umsetzung in einem organischen Lösemittel, vorzugsweise in Methanol, Ethanol, Essigsäureethylester, Diisopropylether, Methyl-tert-butylether, Toluol oder/und chlorierten Lösemitteln, insbesondere in Dichlormethan, erfolgt.

6. Verfahren nach einem der Ansprüchen 1 bis 5, **dadurch gekennzeichnet, dass** das stöchiometrische Verhältnis der Oxoverbindung zur Blausäure und/oder dem Cyaniddonor zwischen 1 : 1 und 1 : 10, vorzugsweise zwischen 1 : 1 un 1 : 4,0 liegt.

7. Verfahren nach einem der Ansprüche 1 bis 6, **dadurch gekennzeichnet, dass** man als Oxoverbindung Aldehyde und/oder Ketone, insbesondere aromatische C₆₋₂₀-Aldehyde, aliphatische C₁₋₂₀-Aldehyde und mit 2 unterschiedlichen Alkyl- und/oder Arylresten substituierte C₄₋₂₀-Ketone einsetzt.

8. Verfahren nach Anspruch 7, **dadurch gekennzeichnet, dass** man als aromatische Aldehyde Benzaldehyd oder substituierte und vorzugsweise mit Alkoxy-, Phenoxy-, Alkyl-, Aryl- oder Halogen-substituierte Benzaldehyde verwendet.

9. Verfahren nach einem der Ansprüche 1 bis 8, **dadurch gekennzeichnet, dass** man als Cyaniddonor Acetoncyanhydrin, Trimethylsilylcyanid und/oder Cyanidsalze, insbesondere Natriumcyanid und Kaliumcyanid einsetzt.

10. Verfahren nach einem der Ansprüche 1 bis 9, **dadurch gekennzeichnet, dass** pro g Oxoverbindung 0,1 g bis 1 000 g, vorzugsweise 1 g bis 50 g Lösemittel zugesetzt werden.

11. Verfahren nach einem der Ansprüche 1 bis 10, **dadurch gekennzeichnet, dass** pro g Oxoverbindung eine Menge an Enzym-haltiger Komponente mit einer Oxynitrilase-Aktivität von 1 U bis 10 000 U, bevorzugt von 20 U bis 2 000 U, zugesetzt wird.

12. Verfahren nach einem der Ansprüche 1 bis 11, **dadurch gekennzeichnet, dass** sich die Oxynitrilase in Zellen und vorzugsweise in gentechnisch veränderten Zellen, in Micellen oder in Mandelkernkleie befindet.

13. Verfahren nach einem der Ansprüche 1 bis 12, **dadurch gekennzeichnet, dass** die molekulargewichtsvergrößerte Oxynitrilase eine bevorzugte Molmasse > 60 000 g/mol und insbesondere > 200 000 g/mol aufweist.

14. Verfahren nach Anspruch 13, **dadurch gekennzeichnet, dass** die Molekulargewichtsvergrößerung durch Quervernetzung der Oxynitrilase, vorzugsweise mit Glutaraldehyd und/oder mit einem Diisocyanat und ggf. einem Trägermaterial erfolgt ist.

15. Verfahren nach Anspruch 13, **dadurch gekennzeichnet, dass** die Molekulargewichtsvergrößerung durch eine Co-Quervernetzung der Oxynitrilase vorzugsweise mit Glutaraldehyd, Chitosan, Polyethylenimin, Polyvinylamin, Gelatine oder beliebigen Mischungen daraus erfolgt ist.

16. Verfahren nach Anspruch 13, **dadurch gekennzeichnet, dass** die Molekulargewichtsvergrößerung durch Flocculation der Oxynitrilase, vorzugsweise mit Polyelektrolyten wie z. B. Polykationen der Reihe Poly(dialkylammoniumchlorid), Chitosan, Diethylaminoethyldextran, Polyanionen der Reihe Polystyrolsulfonsäure, Cellulosesulfat, Sulfaethylcellulose, Dextransulfat, Polyacrylsäure und beliebige Mischungen daraus erreicht wurde.

17. Verfahren nach Anspruch 13, **dadurch gekennzeichnet, dass** die Molekulargewichtsvergrößerung durch eine kovalente Anbindung der Oxynitrilase an anorganische Trägermaterialien, insbesondere an aktiviertes poröses Glas, erzielt wurde.

18. Verfahren nach einem der Ansprüche 13 bis 17, **dadurch gekennzeichnet, dass** die Molekulargewichtsvergrößerung mit der freien Form der Oxynitrilase durchgeführt wurde.

19. Verfahren nach einem der Ansprüche 13 bis 17, **dadurch gekennzeichnet, dass** die Molekulargewichtsvergrößerung der Oxynitrilase in Zellen durchgeführt wurde, vorzugsweise in gentechnisch veränderten Zellen, in Micellen oder in Mandelkernkleie.

20. Verfahren nach einem der Ansprüche 1 bis 19, **dadurch gekennzeichnet, dass** der Geleinschluss und/oder die Gelkomplexierung der Enzym-haltigen Komponente durch Umsetzung mit Polyvinylalkohol und unter Ausbildung von Polyvinylalkohol-Gelen durchgeführt wurde.

21. Verfahren nach einem der Ansprüche 1 bis 19, **dadurch gekennzeichnet, dass** der Geleinschluss und/oder die Gelkomplexierung der Enzym-haltigen Komponente durch Umsetzung mit einer Polycarbamoylsulfonatquelle und unter Ausbildung von Polycarbamoylsulfonat-Gelen durchgeführt wurde.

22. Verfahren nach einem der Ansprüche 1 bis 19, **dadurch gekennzeichnet, dass** der Geleinschluss und/oder die Gelkomplexierung der Enzym-haltigen Komponente durch Kryogelierung, vorzugsweise mit flüssigem Stickstoff durchgeführt wurde.

23. Verfahren nach einem der Ansprüche 20 bis 22, **dadurch gekennzeichnet, dass** der Geleinschluss und/oder die Gelkomplexierung an gentechnisch veränderten Zellen oder Micellen durchgeführt wurde, die eine erhöhte Zellwandpermeabilität aufweisen.

24. Verfahren nach einem der Ansprüche 1 bis 23, **dadurch gekennzeichnet, dass** linsenförmige enzymhaltige Gele eingesetzt werden, vorzugsweise mit einem Durchmesser von mindestens 0,5 mm bis 10 mm und besonders bevorzugt von 3 mm bis 10 mm, sowie einer Dicke von 20 µm bis 5000 µm und besonders bevorzugt von 200 µm bis 2000 µm.

25. Verfahren nach einem der Ansprüche 1 bis 24, **dadurch gekennzeichnet, dass** kugelförmige enzymhaltige Gele eingesetzt werden, vorzugsweise mit einem Durchmesser von ca. 50 µm bis 5000 µm und besonders bevorzugt von 200 µm bis 2000 µm.

## Claims

1. Process for the preparation of optically active cyanhydrins by reaction of oxo compounds with hydrogen cyanide and/or with a cyanide donor and in the presence of an inclusion compound that contains an oxynitrilase of increased molecular weight, **characterised in that** there is used an oxynitrilase which, substantially in two independent steps, has first had its molecular weight increased by crosslinking and has subsequently been converted into a gel inclusion compound and/or a gel complex compound.

2. Process according to claim 1, **characterised in that** the preparation takes place at temperatures of from -10 to +30°C, especially from 5 to 25°C.

3. Process according to either claim 1 or claim 2, **characterised in that** the preparation is carried out at pH values of from 3.0 to 6.0.

4. Process according to any one of claims 1 to 3, **characterised in that** the reaction takes place in an aqueous or water-containing system, preferably in a system from the group water, water/ethanol, water/methanol, water/ethyl acetate, water/diisopropyl ether, water/methyl tert-butyl ether and water/hexane.

5. Process according to any one of claims 1 to 4, **characterised in that** the reaction takes place in an organic solvent, preferably in methanol, ethanol, ethyl acetate, diisopropyl ether, methyl tert-butyl ether, toluene or/and chlorinated solvents, especially in dichloromethane.

6. Process according to any one of claims 1 to 5, **characterised in that** the stoichiometric ratio of the oxo compound to the hydrogen cyanide and/or the cyanide donor is from 1:1 to 1:10, preferably from 1:1 to 1:4.0.

7. Process according to any one of claims 1 to 6, **characterised in that** there are used as the oxo compound aldehydes and/or ketones, especially aromatic C₆₋₂₀-aldehydes, aliphatic C₁₋₂₀-aldehydes, and C₄₋₂₀-ketones substituted by two different alkyl and/or aryl radicals.

8. Process according to claim 7, **characterised in that** there are used as the aromatic aldehydes benzaldehyde or substituted and preferably alkoxy-, phenoxy-, alkyl-, aryl- or halo-substituted benzaldehydes.

9. Process according to any one of claims 1 to 8, **characterised in that** there is used as the cyanide donor acetone cyanhydrin, trimethylsilyl cyanide and/or cyanide salts, especially sodium cyanide and potassium cyanide.

10. Process according to any one of claims 1 to 9, **characterised in that** there are added per g of oxo compound from 0.1 g to 1000 g, preferably from 1 g to 50 g, of solvent.

11. Process according to any one of claims 1 to 10, **characterised in that** there is added per g of oxo compound an amount of enzyme-containing component having an oxynitrilase activity of from 1 U to 10,000 U, preferably from 20 U to 2000 U.

12. Process according to any one of claims 1 to 11, **characterised in that** the oxynitrilase is located in cells and preferably in cells modified by genetic engineering, in micelles or in almond kernel bran.

13. Process according to any one of claims 1 to 12, **characterised in that** the oxynitrilase of increased molecular weight has a preferred molar mass > 60,000 g/mol and especially > 200,000 g/mol.

14. Process according to claim 13, **characterised in that** the increase in molecular weight has been effected by crosslinking of the oxynitrilase, preferably with glutaraldehyde and/or with a diisocyanate and optionally a carrier material.

15. Process according to claim 13, **characterised in that** the increase in molecular weight has been effected by co-crosslinking of the oxynitrilase preferably with glutaraldehyde, chitosan, polyethyleneimine, polyvinylamine, gelatin or any desired mixtures thereof.

16. Process according to claim 13, **characterised in that** the increase in molecular weight has been achieved by flocculation of the oxynitrilase, preferably with polyelectrolytes such as, for example, polycations of the group poly(dialkylammonium chloride), chitosan, diethylaminoethyldextran, polyanions of the group polystyrenesulfonic acid, cellulose sulfate, sulfaethylcellulose, dextran sulfate, polyacrylic acid and any desired mixtures thereof.

17. Process according to claim 13, **characterised in that** the increase in molecular weight has been achieved by covalent binding of the oxynitrilase to inorganic carrier materials, especially to activated porous glass.

18. Process according to any one of claims 13 to 17, **characterised in that** the increase in molecular weight has been carried out with the free form of oxynitrilase.

19. Process according to any one of claims 13 to 17, **characterised in that** the increase in molecular weight of the oxynitrilase has been carried out in cells, preferably in cells modified by genetic engineering, in micelles or in almond kernel bran.

20. Process according to any one of claims 1 to 19, **characterised in that** the gel inclusion and/or the gel complexing of the enzyme-containing component has been carried out by reaction with polyvinyl alcohol and with the formation of polyvinyl alcohol gels.

21. Process according to any one of claims 1 to 19, **characterised in that** the gel inclusion and/or the gel complexing of the enzyme-containing component has been carried out by reaction with a polycarbamoylsulfonate source and with formation of polycarbamoylsulfonate gels.

22. Process according to any one of claims 1 to 19, **characterised in that** the gel inclusion and/or the gel complexing of the enzyme-containing component has been carried out by cryogelation, preferably with liquid nitrogen.

23. Process according to any one of claims 20 to 22, **characterised in that** the gel inclusion and/or the gel complexing has been carried out on genetically engineered cells or micelles having increased cell wall permeability.

24. Process according to any one of claims 1 to 23, **characterised in that** there are used lenticular enzyme-containing gels, preferably having a diameter of at least from 0.5 mm to 10 mm and particularly preferably from 3 mm to 10 mm and a thickness of from 20 µm to 5000 µm and particularly preferably from 200 µm to 2000 µm.

25. Process according to any one of claims 1 to 24, **characterised in that** there are used spherical enzyme-containing gels, preferably having a diameter of approximately from 50 µm to 5000 µm and particularly preferably from 200 µm to 2000 µm.

## Revendications

1. Procédé pour la préparation de cyanhydrines optiquement actives par réaction de composés oxo avec l'acide cyanhydrique et/ou avec un donneur de cyanure et en présence d'un composé d'inclusion qui contient une oxynitrilase de masse moléculaire accrue,
**caractérisé en ce qu'**
on utilise une oxynitrilase dont en premier lieu la masse moléculaire est accrue par réticulation transversale et qui a été ensuite convertie en un composé d'inclusion dans un gel et/ou de complexe de gel, essentiellement en deux étapes indépendantes l'une de l'autre.

2. Procédé selon la revendication 1,
**caractérisé en ce que**
la préparation s'effectue à des températures de réaction de -10 à +30°C, en particulier de 5 à 25°C.

3. Procédé selon la revendication 1 ou 2,
**caractérisé en ce qu'**
on effectue la préparation à des pH de 3,0 à 6,0.

4. Procédé selon l'une quelconque des revendications 1 à 3,
**caractérisé en ce qu'**
on effectue la réaction dans un système aqueux ou contenant de l'eau, de préférence dans un système de la série eau, eau/éthanol, eau/ méthanol, eau/acétate d'éthyle, eau/éther diisopropylique, eau/oxyde de méthyle et de tert-butyle et eau/hexane.

5. Procédé selon l'une quelconque des revendications 1 à 4,
**caractérisé en ce que**
la réaction s'effectue dans un solvant organique, de préférence dans du méthanol, de l'éthanol, de l'acétate d'éthyle, de l'éther diisopropylique, de l'oxyde de méthyle et de tert-butyle, du toluène et/ou des solvants chlorés, en particulier dans du dichlorométhane.

6. Procédé selon l'une quelconque des revendications 1 à 5,
**caractérisé en ce que**
le rapport stoechiométrique du composé oxo à l'acide cyanhydrique et/ou au donneur de cyanure est compris entre 1:1 et 1:10, de préférence entre 1:1 et 1:4,0.

7. Procédé selon l'une quelconque des revendications 1 à 6,
**caractérisé en ce qu'**
on utilise comme composé oxo des aldéhydes et/ou des cétones, en particulier des aldéhydes aromatiques en C₆₋₂₀, des aldéhydes aliphatiques en C₁₋₂₀ et des cétones en C₄₋₂₀ substituées par deux radicaux alkyle et/ou aryle différents

8. Procédé selon la revendication 7,
**caractérisé en ce qu'**
on utilise comme aldéhydes aromatiques le benzaldéhyde ou des benzaldéhydes substitués et de préférence substitués par des halogènes ou des groupes alcoxy, phénoxy, alkyle ou aryle.

9. Procédé selon l'une quelconque des revendications 1 à 8,
**caractérisé en ce qu'**
on utilise comme donneur de cyanure l'acétone-cyanhydrine, le cyanure de triméthylsilyle et/ou des cyanures, en particulier le cyanure de sodium et le cyanure de potassium.

10. Procédé selon l'une quelconque des revendications 1 à 9,
**caractérisé en ce qu'**
on ajoute par g de composé oxo de 0,1 g à 1 000 g, de préférence de 1 g à 50 g de solvant.

11. Procédé selon l'une quelconque des revendications 1 à 10,
**caractérisé en ce qu'**
on ajoute par g de composé oxo une quantité de composant contenant une enzyme ayant une activité oxynitrilase de 1 U à 10 000 U, de préférence de 20 U à 2 000 U.

12. Procédé selon l'une quelconque des revendications 1 à 11,
**caractérisé en ce que**
l'oxynitrilase se trouve dans des cellules et de préférence dans des cellules génétiquement modifiées, dans des micelles ou dans de la farine d'amande.

13. Procédé selon l'une quelconque des revendications 1 à 12,
**caractérisé en ce que**
l'oxynitrilase dont la masse moléculaire a été accrue présente une masse moléculaire préférée > 60 000 g/mole et en particulier > 200 000 g/mole.

14. Procédé selon la revendication 13,
**caractérisé en ce que**
l'accroissement de masse moléculaire a été effectué par réticulation transversale de l'oxynitrilase, de préférence avec du glutaraldéhyde et/ou avec un diisocyanate et éventuellement un matériau de support.

15. Procédé selon la revendication 13,
**caractérisé en ce que**
l'accroissement de masse moléculaire a été effectué par une co-réticulation transversale de l'oxynitrilase, de préférence avec du glutaraldéhyde, du chitosane, de la polyéthylène-imine, de la polyvinylamine, de la gélatine ou des mélanges quelconques de ceux-ci.

16. Procédé selon la revendication 13,
**caractérisé en ce que**
l'accroissement de masse moléculaire a été effectué par floculation de l'oxynitrilase, de préférence avec des polyélectrolytes, comme par exemple des polycations choisis parmi un poly(chlorure de dialkylammonium), le chitosane, le diéthylaminoéthyldextrane, des polyanions choisis parmi l'acide polytyrènesulfonique, le sulfate de cellulose, la sulfaéthylcellulose, le sulfate de dextrane, le poly(acide acrylique) et des mélanges quelconques de ceux-ci.

17. Procédé selon la revendication 13,
**caractérisé en ce que**
l'accroissement de masse moléculaire a été obtenu par une fixation par liaison covalente de l'oxynitrilase sur des matériaux de support non organiques, en particulier sur du verre poreux activé.

18. Procédé selon l'une quelconque des revendications 13 à 17,
**caractérisé en ce que**
l'accroissement de masse moléculaire a été effectué avec la forme libre de l'oxynitrilase.

19. Procédé selon l'une quelconque des revendications 13 à 17,
**caractérisé en ce que**
l'accroissement de masse moléculaire de l'oxynitrilase a été effectué dans des cellules, de préférence dans des cellules génétiquement modifiées, dans des micelles ou dans de la farine d'amande.

20. Procédé selon l'une quelconque des revendications 1 à 19,
**caractérisé en ce que**
l'inclusion dans un gel et/ou la complexation de gel du composant contenant l'enzyme ont été effectuées par réaction avec du poly(alcool vinylique) et avec formation de gels de poly(alcool vinylique).

21. Procédé selon l'une quelconque des revendications 1 à 19,
**caractérisé en ce que**
l'inclusion dans un gel et/ou la complexation de gel du composant contenant l'enzyme ont été effectuées par réaction avec une source de polycarbamoylsulfonate et avec formation de gels de polycarbamoylsulfonate.

22. Procédé selon l'une quelconque des revendications 1 à 19,
**caractérisé en ce que**
l'inclusion dans un gel et/ou la complexation de gel du composant contenant l'enzyme ont été effectuées par cryogélification, de préférence avec de l'azote liquide.

23. Procédé selon l'une quelconque des revendications 20 à 22,
**caractérisé en ce que**
l'inclusion dans un gel et/ou la complexation de gel a été effectuée sur des cellules génétiquement modifiées ou des micelles qui présentent une perméabilité accrue de la membrane cellulaire.

24. Procédé selon l'une quelconque des revendications 1 à 23,
**caractérisé en ce qu'**
on utilise des gels lenticulaires contenant une enzyme, de préférence à particules d'un diamètre d'au moins 0,5 mm à 10 mm et de façon particulièrement préférée de 3 mm à 10 mm, ainsi que d'une épaisseur de 20 µm à 5 000 µm, et de façon particulièrement préférée de 200 µm à 2 000 µm.

25. Procédé selon l'une quelconque des revendications 1 à 24,
**caractérisé en ce qu'**
on utilise des gels contenant une enzyme, à particules sphériques de préférence ayant un diamètre d'environ 50 µm à 5 000 µm et de façon particulièrement préférée de 200 µm à 2 000 µm.
